# EUROPÄISCHE PATENTSCHRIFT

(11) **EP 0 459 242 B1**
(45) Veröffentlichungstag und Bekanntmachung des Hinweises auf die Patenterteilung: **11.10.1995**
(21) Anmeldenummer: 91108000.0
(22) Anmeldetag: 17.05.1991
(51) Int. Cl.: C07C 43/205, C07C 41/16

(54) **Verfahren zur Herstellung von Phenolethern**
Process for the preparation of phenolethers
Procédé pour la préparation d'éthers phénoliques

(30) Priorität: 31.05.1990 DE 4017524
(43) Veröffentlichungstag der Anmeldung: 04.12.1991
(73) Patentinhaber: BASF Aktiengesellschaft, 67063 Ludwigshafen (DE)
(72) Erfinder: Houben, Jochen, Dr., W-6520 Worms 26 (DE); Weber, Wilhelm, Dr., W-6730 Neustadt (DE)

(56) Entgegenhaltungen:
- EP-A- 0 071 182
- HOUBEN-WEYL, "Methoden der organischen Chemie", 1965, Georg Thieme Verlag, Stuttgart, DE, Band VI/3, 1965, S. 62-66
- JOURNAL FÜR PRAKTISCHE CHEMIE, Bd. 35, 1967, Seiten 110 - 112; M. GEISERT et al: "Über eine einfache und ergiebige Synthese des 2,4,6-Trimethoxyanilins"
- PATENT ABSTRACTS OF JAPAN, vol. 11, no. 24 (C-399)(2471), 23. Januar 1987; & JP-A-61 197 538
- INDUSTRIAL AND ENGINEERING CHEMISTRY, Am. Chem. Society, Bd. 22, Nr. 1, 1. Januar 1930, Columbus, US, Seiten 34 - 36; HARRY F. LEWIS ET AL: "Methylation of Phenol by Dimethyl Sulfate"

## Beschreibung

Die Vorliegende Erfindung betrifft ein Verfahren zur Herstellung von Phenolethern durch Umsetzung von Phenolen mit Alkylsulfaten bei Temperaturen von 0 bis +140°C.

Aus Berichte der deutschen chem. Ges. 33, 2476-2477 (1900) ist die Veretherung von Phenolen bekannt. Ferner ist aus Annalen der Chemie, 204-212 (1905) bekannt, daß auch die zweite Methylgruppe des Dimethylsulfats in die Reaktion eingebracht werden kann. Bei dem dort verwendeten Verfahren wird die gesamte Menge der Hilfsbase vor dem Erhitzten zugegeben, wodurch der pH-Wert der Reaktionsmischung von Anfang an deutlich über 12 liegt. Bei diesem pH erhält man Ausbeuten von bis zu 80 % des aromatischen Methylethers.

Aus der RO-A-62678 und J. prakt. Chem., Bd. 35, 110-112 (1967) sind Umsetzungen von Phenolen mit Dialkylsulfaten bei pH-Werten von 7 bis 8,5 bzw. 8 bis 9 bekannt, jedoch wurde nur eine Alkylgruppe des Alkylierungsmittels zur Veretherung verwendet.

Der vorliegenden Erfindung lag daher die Aufgabe zugrunde, den zuvor genannten Nachteilen abzuhelfen.

Demgemäß wurde ein verbessertes Verfahren zur Herstellung von Phenolethern durch Umsetzung von Phenolen mit Alkylsulfaten bei Temperaturen von 0 bis +140°C gefunden, welches dadurch gekennzeichnet ist, daß man die Umsetzung in wäßrigen Medium
a) im Falle von Dialkylsulfaten zu Phenol in einem Molverhältnis von 0,5:1 bis 0,8:1, in einem pH-Bereich von 7 bis 10 und anschließend in einem pH-Bereich von 10 bis 12 oder
b) im Falle von Monoalkylsulfaten zu Phenol in einem Molverhältnis von 1:1 bis 1,6:1, in einem pH-Bereich von 10 bis 12 durchführt.

Die Umsetzung der Alkylsulfate mit den Phenolen läßt sich allgemein wie folgt durchführen:
Das entsprechende Phenol wird zusammen mit 0,2 bis 0,5 Äquivalenten Lauge pro phenolischer OH-Gruppe in einer zum Lösen gerade ausreichenden Menge Wassers bei 30 bis 60°C vorgelegt. Bei oxidationsempfindlichen Phenolen, wie z.B. Brenzkatechin oder Hydrochinon sollte unter Luftausschluß gearbeitet werden.

Sodann werden 0,5 bis 0,8 Äquivalente Dialkylsulfat in dem Maße zugetropft, wie die Reaktionswärme durch Außenkühlung abgeführt werden kann, wobei eine Innentemperatur von 60°C nicht überschritten werden sollte.

Während der Dialkylsulfatzugabe und auch danach wird der pH durch kontinuierliche Zugabe von Lauge auf einen Wert zwischen 7 und 10 gehalten. Nach der Zugabe wird auf eine Temperatur zwischen 90 und 140°C aufgeheizt, wobei höhere Temperaturen stets vorzuziehen sind, da sie zu kürzeren Reaktionszeiten und besseren Ausbeuten führen. In Abhängigkeit von der Dissoziationskonstante des Phenols wird durch Zugabe von Lauge ein pH-Wert zwischen 10 und 12 eingestellt.

Je nach Reaktionstemperatur und eingesetztem Phenol dauert diese zweite Stufe zwischen 1 und 24 Stunden.

Anschließend wird soviel Wasser zugegeben, daß sich das gebildete Sulfat gerade löst und bei einer Temperatur von 20 bis 50°C werden die Phasen getrennt. Die Wertproduktphase wird durch Waschen mit Lauge von nicht umgesetzten Phenol befreit und bei besonderen Reinheitsanforderungen anschließend noch destilliert.

Beim Einsatz eines Monoalkylsulfats legt man das Phenol und 1,1 bis 1,6 Äquivalente Monoalkylsulfat in einer zum Lösen gerade ausreichenden Menge Wassers vor und rührt bei 90 bis 140°C bis zur Vollständigkeit der Reaktion. Dabei hält man durch Laugenzugabe den pH auf einem Wert zwischen 10 und 12. Man arbeitet wie oben angegeben auf.

Als Laugen eignen sich z.B. Natronlauge und Kalilauge.

Als Phenole eignen sich unsubstituiertes Phenol oder substituierte Phenole wie ein- bis fünffach substituierte Phenole, die durch C₁- bis C₈-Alkyl, vorzugsweise C₁- bis C₄-Alkyl, ein- bis dreifach substituiert sind, wie 2-Methylphenol, 3-Methylphenol, 4-Methylphenol, 2,3-Dimethylphenol, 2,4-Dimethylphenol, 2,5-Dimethylphenol, 2,6-Dimethylphenol, 3,4-Dimethylphenol, 3,5-Dimethylphenol und 2,4,6-Trimethylphenol oder auch mit anderen Resten wie Hydroxy, C₁- bis C₄-Alkoxy, Halogen oder NO₂-Gruppen substituierte Phenole in Frage, außer solchen Phenolen, die in o- und/oder p-Stellung zum Halogenatom zusätzlich eine Nitrogruppe tragen. Darunter fallen Brenkatechin, Resorcin, Guajacol, o-Chlorphenol, p-Chlorphenol, p-Nitrophenol und p-Hydroxybenzolsulfonsäure.

Ebenso kommen Phenole in Betracht, die neben einem Alkylrest noch eine oder zwei andere Gruppen tragen, wie z.B. 4-Methylbrenzkatechin, 3,5-t-Butylhydrochinon, ebenso kommen mehrkernige aromatische Systeme, wie α- oder β-Hydroxynaphthaline oder 1,4-Dihydroxynaphthaline oder alkylsubstituierte Naphthole in Frage.

Unter den Phenolethern werden Methylether und die Ethylether wie p-Methylanisol, Veratrol, Hydrochinondimethylether und 1-Methoxynaphthalin bevorzugt.

Als Dialkylsulfate eignen sich besonders Dimethyl- und Diethylsulfat, bevorzugt Dimethylsulfat.

Als Monoalkylsulfate eignen sich die Alkalisalze des Schwefelsäuremonomethylesters und des Schwefelsäuremonoethylesters, bevorzugt die Natrium- und Kaliumsalze, besonders bevorzugt das Natriumsalz des Schwefelsäuremonomethylesters.

Das Molverhältnis Dialkylsulfat zu Phenol liegt zwischen 0,5:1 und 0,8:1, bevorzugt zwischen 0,5:1 und 0,7:1, besonders bevorzugt zwischen 0,5:1 und 0,6:1.

Das Molverhältnis Monoalkylsulfat zu Phenol liegt zwischen 1:1 und 1,6:1, bevorzugt zwischen 1:1 und 1,4:1, besonders bevorzugt zwischen 1:1 und 1,2:1.

Die Reaktion wird in wäßrigem Medium bei Temperaturen von 0 bis 140°C so durchgeführt, daß während der gesamten Reaktion das Wasser nicht aus dem System entfernt werden muß.

Dabei wird die 1. Stufe der Reaktion, die Umsetzung des Dialkylsulfats bei Temperaturen von 20 bis 80°C, bevorzugt aber von 40 bis 60°C im pH-Bereich von 7 bis 10 durchgeführt, während die Umsetzung des Schwefelsäuremonoalkylethers bei Temperaturen von 80 bis 140°C, bevorzugt aber bei 95 bis 120°C im pH-Bereich von 10 bis 12, bevorzugt 10 bis 11,75, durchgeführt wird.

Die Phenolether sind Zwischenprodukte für Kunststoffe und für den Pflanzenschutz.

### Beispiele

### Beispiel 1

200 ml Wasser und 40 g (0,5 Mol) 50 %ige Natronlauge werden vorgelegt. Bei 30 bis 40°C werden 216 g (2 Mol) p-Kresol zugetropft. Der pH-Wert beträgt dann ca. 9,5. 151,2 g (1,2 Mol) Dimethylsulfat werden im Laufe von 1 Stunde zugetropft. Dabei wird der pH von 9,5 durch Zugabe von 50 %iger Natronlauge gehalten. Nachdem die Dimethylsulfat-Zugabe beendet ist, wird zum Rückfluß erhitzt und der pH auf 10,5 hochgestellt. Es wird 4 Stunden am Rückfluß gelassen und dabei der pH durch Zugabe von 50 %iger Natronlauge auf 10,5 gehalten. Insgesamt werden außer den vorgelegten 40 g Natriumhydroxid weitere 140 g (1,75 Mol) 50 %ige Natronlauge benötigt. Nach den 4 Stunden Rückfluß werden 110 g Wasser zugesetzt und auf 30 bis 40°C abgekühlt. Die Phasen werden getrennt. Die untere Phase wird verworfen. Die obere Phase wird einmal mit 40 g 10 %iger Natronlauge und einmal mit 40 g 20 %iger Natronlauge gewaschen.

Man erhält 228 g (93,5 %) p-Methylanisol (Reinheit laut GC: 99,7 %).

### Beispiel 2

100 ml Wasser und 25 g (0,3125 Mol) 50 %ige Natronlauge wurden vorgelegt und bei 40°C 108 g (1 Mol) p-Kresol zugesetzt. Innerhalb 20 Minuten wurden bei 50 bis 55°C 63 g (0,5 Mol) Dimethylsulfat zugetropft. Gleichzeitig beginnend wurden innerhalb 2 Stunden 60 g (0,75 Mol) 50 %ige Natronlauge zugetropft. Sobald alles Dimethylsulfat zugegeben war, wurde zum Rückfluß aufgeheizt und 6 Stunden am Rückfluß belassen. Anschließend wurden 80 g Wasser zugesetzt und auf 45°C abgekühlt. Nach Phasentrennung wurde mit 20 g 10 %iger Natronlauge gewaschen.

Man erhält 110 g (90 %) p-Methylanisol (Reinheit: 99,6 %).

### Beispiel 3

200 ml Wasser und 70 g (0,875 Mol) 50 %ige Natronlauge werden vorgelegt. Unter Kühlung werden 188 g (2 Mol) Phenol zugegeben und anschließend 145 g (1,15 Mol) Dimethylsulfat im Laufe von 15 Minuten bei maximal 60°C zugetropft. Anschließend wird der Ansatz zum Sieden erhitzt und 7 Stunden am Rückfluß belassen. Während der Zugabe des Dimethylsulfates und auch danach wird der pH-Wert durch Zugabe von Natronlauge (insgesamt 126 g ≙ 1,575 Mol) auf 11,0 eingestellt. Es wird wie üblich aufgearbeitet und man erhält 198 g (91,7 %) Anisol.

### Beispiel 4

Beispiel 1 wird wiederholt, aber die zweite Reaktionsstufe wird nicht unter Normaldruck bei 100°C, sondern in einem Rührautoklaven unter Eigendruck bei 120°C ausgeführt. Die Reaktion ist bereits nach 2 statt nach 4 Stunden beendet.

Man erhält 240 g (98,5 %) p-Methylanisol (Reinheit 99,85 %).

### Beispiel 5

100 ml Wasser und 140 g (1,75 Mol) 50 %iger Natronlauge werden unter Stickstoff vorgelegt. Es werden unter Rühren 220,2 g (2 Mol) Brenzkatechin eingetragen und mit 100 ml Wasser nachgespült. Dabei stellt sich ein pH von 9,7 ein. Unter Kühlung werden innerhalb von 60 Minuten bei einer Temperatur von 45 bis 50°C 302,49 g (2,4 Mol) Dimethylsulfat zugetropft, wobei der pH durch Zugabe von insgesamt 65 g (0,8125 Mol) 50 %iger NaOH zwischen 9 und 10 gehalten wird.

Anschließend wird binnen einer Stunde zum Sieden erhitzt und 12 Stunden am Rückfluß belassen. Während dieser Zeit wird der pH durch Zugabe von Natronlauge auf einem Wert zwischen 11 und 11,5 gehalten. Abschließend wird noch ein Teil Natronlauge eingetragen, so daß die gesamte Laugezugabe während der zweiten Stufe 195 g (2,44 Mol) 50 %iger NaOH beträgt.

Es wird auf 35°C abgekühlt, mit 500 ml Wasser verdünnt, die Phasen getrennt und die Wertproduktphase zweimal mit je 60 g 15 %iger NaOH gewaschen.

Die Destillation bei 23 bis 25 mbar ergibt 262,4 g (95 %) Veratrol mit einem Siedepunkt von 92°C und einer Reinheit von 99,72 %. Das Produkt enthält kein 4-Methylveratrol.

## Patentansprüche

1. Verfahren zur Herstellung von Phenolethern durch Umsetzung von Phenolen mit Alkylsulfaten bei Temperaturen von 0 bis +140°C, dadurch gekennzeichnet, daß man die Umsetzung in wäßrigem Medium
a) im Falle von Dialkylsulfaten zu Phenol in einem Molverhältnis von 0,5:1 bis 0,8:1, in einem pH-Bereich von 7 bis 10 und anschließend in einem pH-Bereich von 10 bis 12 oder
b) im Falle von Monoalkylsulfaten zu Phenol in einem Molverhältnis von 1:1 bis 1,6:1, in einem pH-Bereich von 10 bis 12 durchführt.

2. Verfahren nach Anspruch 1, dadurch gekennzeichnet, daß man die Umsetzung in wäßrigem Medium
a) im Falle von Dialkylsulfaten zu Phenol in einem Molverhältnis von 0,5:1 bis 0,8:1, in einem pH-Bereich von 7 bis 10 und anschließend in einem pH-Bereich von 10 bis 11,75 oder
b) im Falle von Monoalkylsulfaten zu Phenol in einem Molverhältnis von 1:1 bis 1,6:1, in einem pH-Bereich von 10 bis 11,75 durchführt.

3. Verfahren nach Anspruch 1, dadurch gekennzeichnet, daß man ein Molverhältnis
a) im Falle von Dialkylsulfaten zu Phenol von 0,5:1 bis 0,7:1 oder
b) im Falle von Monoalkylsulfaten zu Phenol von 1:1 bis 1,4:1
einhält.

## Claims

1. A process for preparing a phenol ether by reacting a phenol with an alkyl sulfate at from 0 to +140°C, which comprises carrying out the reaction in aqueous medium
a) in the case of dialkyl sulfates to phenol in a molar ratio of from 0.5:1 to 0.8:1, at a pH in the range from 7 to 10 and subsequently at a pH in the range from 10 to 12 or
b) in the case of monoalkyl sulfates to phenol in a molar ratio of from 1:1 to 1.6:1, at a pH in the range from 10 to 12.

2. A process as claimed in claim 1, wherein the reaction is carried out in aqueous medium
a) in the case of dialkyl sulfates to phenol in a molar ratio of from 0.5:1 to 0.8:1, at a pH in the range from 7 to 10 and subsequently at a pH in the range from 10 to 11.75 or
b) in the case of monoalkyl sulfates to phenol in a molar ratio of from 1:1 to 1.6:1, at a pH in the range from 10 to 11.75.

3. A process as claimed in claim 1, wherein the molar ratio is
a) in the case of dialkyl sulfates to phenol from 0.5:1 to 0.7:1 or
b) in the case of monoalkyl sulfates to phenol from 1:1 to 1.4:1.

## Revendications

1. Procédé de préparation d'éthers phénoliques par la réaction de phénols avec des sulfates d'alkyle à des températures de 0 à +140°C, caractérisé en ce que l'on entreprend la réaction en milieu aqueux
a) dans le cas de sulfates de dialkyle et du phénol en un rapport molaire de 0,5:1 à 0,8:1, dans une plage de pH de 7 à 10 et ensuite dans une plage de pH de 10 à 12, ou
b) dans le cas de sulfates de monoalkyle et du phénol dans un rapport molaire de 1:1 à 1,6:1, dans une plage de pH de 10 à 12.

2. Procédé suivant la revendication 1, caractérisé en ce que l'on entreprend la réaction en milieu aqueux
a) dans le cas de sulfates de dialkyle et du phénol dans le rapport molaire de 0,5:1 à 0,8:1, dans une plage de pH de 7 à 10 et ensuite dans une plage de pH de 10 à 11,75, ou
b) dans le cas de sulfates de monoalkyle et de phénol dans le rapport molaire de 1:1 à 1,6:1, dans la plage des pH de 10 à 11,75.

3. Procédé suivant la revendication 1, caractérisé en ce que l'on maintient un rapport molaire
a) dans le cas des sulfates de dialkyle et du phénol de 0,5:1 à 0,7:1, ou
b) dans le cas de sulfates de monoalkyle et du phénol de 1:1 à 1,4:1.
